# EUROPEAN PATENT SPECIFICATION

(11) **EP 2 761 994 B1**
(45) Date of publication and mention of the grant of the patent: **02.11.2016**
(21) Application number: 14153616.9
(22) Date of filing: 03.02.2014
(51) Int. Cl.: A01G 1/00, A01G 7/04, A01G 31/00, A01G 33/00, A01H 3/02

(54) **Plant cultivation method and plant cultivation apparatus**
Pflanzenzuchtverfahren und Pflanzenzuchtanlage
Procédé de culture de plantes et appareil de culture de plantes

(30) Priority: 04.02.2013 JP 2013019931; 31.01.2014 JP 2014017338
(43) Date of publication of application: 06.08.2014
(73) Proprietor: SHOWA DENKO K.K., Tokyo 105-8518 (JP)
(72) Inventor: Takeuchi, Ryouichi, Tokyo, 105-8518 (JP); Ara, Hironori, Tokyo, 105-8518 (JP); Suzuki, Hiroshi, Tokyo, 105-8518 (JP)
(74) Representative: Strehl Schübel-Hopf & Partner

(56) References cited:
- EP-A1- 1 479 286
- JP-A- H06 276 858
- JP-A- H08 103 167
- US-A1- 2012 043 907
- AURÉLIO FAJAR TONETTO ET AL: "Effects of irradiance and spectral composition on the establishment of macroalgae in streams in southern Brazil", ANNALES DE LIMNOLOGIE - INTERNATIONAL JOURNAL OF LIMNOLOGY, vol. 48, no. 4, 1 January 2012 (2012-01-01), pages 363-370, XP055122689, ISSN: 0003-4088, DOI: 10.1051/limn/2012027
- KASPERBAUER MICHAEL J ET AL: "Morphogenic light reflected to developing cotton leaves affects insect-attracting terpene concentrations", CROP SCIENCE: A JOURNAL SERVING THE INTERNATIONAL COMMUNITY OF CROP SCIENTISTS, CROP SCIENCE SOCIETY OF AMERICA, US, vol. 44, no. 1, 1 January 2004 (2004-01-01), pages 198-203, XP009178437, ISSN: 0011-183X

## Description

### BACKGROUND OF THE INVENTION

### Field of the Invention

The present invention relates to a plant cultivation method and a plant cultivation apparatus. More specifically, the present invention relates to a plant cultivation method and a plant cultivation apparatus for promoting growth of a plant by irradiating the plant with artificial light and solar light.

Priority is claimed on Japanese Patent Application No. 2013-019931, filed on February 4, 2013 and Japanese Patent Application No.2014-017338, filed on January 31, 2014.

### Description of Related Art

In the related art, there is a technology that promotes the raising of seedlings by irradiating the plant seedlings with artificial light in plant cultivation. It is possible to shorten a cultivation period and to increase the number of harvesting times at the same place by promoting growth of a plant. In addition, it is possible to increase the amount of harvested crop if the plant can grow larger even under the same cultivation period conditions.

As a method of plant cultivation using artificial light irradiation, for example, Japanese Unexamined Patent Application, First Publication No. H6-276858 discloses an apparatus that irradiates a plant with light configured to alternatingly irradiate the plant with green light and white light. The irradiation apparatus is configured to switch conditions between day and night by alternatingly irradiating a plant with green light having a wavelength range between 500 nm and 570 nm and white light having a wavelength range between 300 nm and 800 nm and grows the plant by facilitating an operation of translocation of a plant.

In addition, for example, Japanese Unexamined Patent Application, First Publication No. H8-103167 discloses a light source for plant cultivation that irradiates a plant with light energy for culturing, growing, cultivating, and tissue-culturing a plant by simultaneously or alternatingly turning on a light-emitting diode that emits blue light (400 nm to 480 nm) and a light-emitting diode that emits red light (620 nm to 700 nm). The light source for plant cultivation is designed to cultivate a plant with good energy efficiency by irradiating a plant only with light having a wavelength that corresponds to a light-absorption peak of chlorophyll (near 450 nm and near 660 nm). It is considered that the alternating irradiation with the blue light and the red light indicates flashing irradiation with a high frequency in Japanese Unexamined Patent Application, First Publication No. H8-103167 (refer to paragraph [0006] of the document).

### SUMMARY OF THE INVENTION

In the related art, when cultivating a plant using irradiation with artificial light, there is a case where solar light is also used in addition to the artificial light in order to improve energy efficiency. Specifically, for example, there is a method of cultivating a plant by setting up illumination equipment for irradiating a plant with artificial light within a plastic greenhouse and by using the artificial light and solar light introduced into the plastic greenhouse.

However, in the technology of the related art, it is impossible to obtain a sufficient effect for promoting growth of a plant when cultivating the plant using the artificial light and the solar light in combination.

Objects of the present invention are to solve the above-described problem and to provide a plant cultivation method and a plant cultivation apparatus that can sufficiently promote the growth of a plant by irradiating a plant with the artificial light and the solar light and can obtain excellent energy efficiency.

The present inventors have carried out intensive studies to solve the above-described problem.

As a result, it was found that it is possible to sufficiently promote the growth of a plant by irradiating a plant with the artificial light and the solar light by independently performing a sequence of irradiating a plant with solar light, a sequence of irradiating the plant with red light, and a sequence of irradiating the plant with blue light, within a certain period of time, and it is possible to obtain excellent energy efficiency, to conceive the present invention.

That is, the present invention relates to the following.
(1) According to an aspect of the present invention, there is provided a plant cultivation method including: a sequence of irradiating a plant with sunlight; a sequence of irradiating the plant with red light; and a sequence of irradiating the plant with blue light, in which the sequences are performed independently within a certain period of time.
(2) In the aspect stated in the above (1), the sequence of irradiating the plant with red light may be performed subsequently to the sequence of irradiating the plant with sunlight.
(3) In the aspect stated in the above (1), the sequence of irradiating the plant with blue light may be performed subsequently to the sequence of irradiating the plant with sunlight.
(4) In the aspect stated in the above (1), the sequence of irradiating the plant with solar light may be performed subsequently to the sequence of irradiating the plant with red light.
(5) In the aspect stated in the above (1), the sequence of irradiating the plant with sunlight may be performed subsequently to the sequence of irradiating the plant with blue light.

In the present invention, at least leafy vegetables, fruit trees, grains, and algae are regarded as the "plant". In addition, phytoplankton such as green algae or mosses are also widely regarded as the "plant" referred to in the present invention.

In the plant cultivation method of an aspect of the present invention, it is possible to promote the growth of a plant by irradiating a plant with the artificial light and the solar light and to obtain excellent energy efficiency by independently performing a sequence of irradiating a plant with solar light, a sequence of irradiating the plant with red light, and a sequence of irradiating the plant with blue light within a certain period of time.

### DETAILED DESCRIPTION OF THE INVENTION

Hereinafter, a preferable embodiment of the present invention is described with an example. Note that the embodiment described below illustrates an example of a typical embodiment of the present invention and this does not limit the scope of the present invention.

### [Plant Cultivation Apparatus]

A plant cultivation apparatus of the present embodiment can execute a plant cultivation method of the present embodiment to be described later. The plant cultivation apparatus of the present embodiment includes a region in which a plant is irradiated with solar light, that is, sunlight, a light irradiation unit that irradiates a plant with artificial light, and a control unit that controls the light irradiation unit.

It is preferable that the region in which a plant is irradiated with solar light is in a position to irradiate a plant to be cultivated directly or indirectly irradiated with solar light. Examples of the region include an outdoor or indoor region capable of putting a plant and preferably a region in which solar light is introduced in a cultivation room provided with a plant to be cultivated.

It is preferable that the cultivation room to be used in the present embodiment be designed such that the temperature and the moisture of the cultivation room can be controlled to be within a predetermined range depending on air conditioners so as to improve the effect for promoting growth of a plant. Furthermore, it is preferable that the cultivation room be designed such that the concentration of carbon dioxide can be controlled to be within a predetermined range.

Examples of means for introducing solar light in the cultivation room include a window or a light guide tube of solar light formed of translucent material installed in a wall and/or a ceiling in a case where a wall and/or a ceiling that separates the cultivation room from the outdoors is formed of a material having a light-shielding property. In addition, Examples thereof also include a portion of a wall and/or ceiling formed of a translucent material in a case where part or all of a wall and/or a ceiling that separate the cultivation room from the outdoor is formed of a translucent material such as a sheet-shaped translucent resin or a plate-shaped translucent resin.

The light irradiation unit irradiates a plant with artificial light including red light and/or blue light, that is, at least one of the red light and blue light, and it is preferable that the light irradiation unit irradiate a plant with artificial light only when solar light is insufficient in order to improve energy efficiency.

Examples of the red light emitted from the light irradiation unit include light having a wavelength between 600 nm and 730 nm, and red light having a wavelength between 645 nm and 670 nm as a center wavelength is suitably used. Examples of blue light emitted from the light irradiation unit include light having a wavelength between 400 nm and 515 nm, and blue light having a wavelength of 450 nm as a center wavelength is suitably used. The red light and the blue light can be set to have a predetermined wavelength range by setting the above-described wavelengths as center wavelengths. Examples of the wavelength range include, in a case of blue light, 450 nm ± 30 nm, preferably 450 nm ± 20 nm, more preferably, 450 nm ± 10 nm.

The light irradiation unit includes a light source of red light and a light source of blue light. It is possible to use conventionally known ones as light sources of red light and blue light. As the light sources, specifically, it is possible to use a light-emitting diode (LED) element, a laser diode (LD) element, electroluminescence (EL) element, a straight tube type or compact type fluorescent lamp, an electric bulb type fluorescent lamp, a high-pressure discharge lamp, a metal halide lamp, or the like in which it is easy to select a wavelength and that emits light having a large proportion of light energy of an effective wavelength range. In a case where the EL element is used as a light source, an organic EL element may be used or an inorganic EL element may be used.

Among the above-described light sources, in particular, a photosemiconductor element such as a light-emitting diode (LED) or a laser diode (LD) is small in size, has a long life, emits light at a specific wavelength depending on materials, has good energy efficiency because there is no unnecessary heat radiation, and does not cause any damage such as leaf scorching even when closely irradiating a plant with light. For this reason, in a case where the photosemiconductor element is used as a light source, it is possible to obtain excellent energy efficiency with low power consumption and to obtain a space-saving light irradiation unit compared to other light sources.

As a light-emitting diode used as a light source of red light, for example, there is an aluminum-gallium-indium-phosphide light-emitting diode (gallium-phosphide substrate, 660 nm of a red wavelength) which has been sold under product number HRP-350F from SHOWA DENKO K.K., or the like.

In addition, as a light-emitting diode used as a light source of blue light, there is a light-emitting diode which has been generally sold as an indium-gallium-nitride light-emitting diode (450 nm of wavelength), or the like.

As the light irradiation unit using the photosemiconductor element as a light source, for example, a light irradiation unit including a surface mount device (SMD) line light source linearly formed of a plurality of SMDs in which a red photosemiconductor element and a blue photosemiconductor element are mounted in combination; a monochromatic line light source where only one of the red photosemiconductor element or the blue photosemiconductor element is linearly formed or formed into a planar shape; or both a monochromatic line light source for red light and a monochromatic line light source for blue light are provided.

The light irradiation unit may have a combination of the light source described above and an optical filter for selectively using red light and/or blue light.

The control unit controls the light irradiation unit to independently perform a step of irradiating a plant with red light and a step of irradiating the plant with blue light within a certain period of time (Shigyo method).

The method, which includes a sequence (process) of irradiating a plant with red light, a sequence (process) of irradiating a plant with blue light, wherein the sequences are performed independently in fixed time periods (within a certain period time), is sometimes called Shigyo Method.

The control unit provided in the plant cultivation apparatus of the present embodiment independently turns on and off the light source of red light and the light source of blue light by supplying a predetermined electric current to the light source of red light and the light source of blue light. In addition, in the present embodiment, it is preferable that the light emission intensity of red light to blue light from the light irradiation unit be controlled by the control unit adjusting the amount of electric current supplied to the light source of red light and the light source of blue light.

The control unit can be configured using a customized computer. Specifically, for example, the control unit can adjust the size of the driving current of a light source and change the light emission intensity of red light and/or blue light and the irradiation time based on a control pattern which is held and stored in a memory device such as a memory or a hard disk in advance. In addition, the control unit can change the wavelength range of emitted light by switching a plurality of light sources that radiate light in a different wavelength range based on the control pattern stored in the memory device.

### [Plant Cultivation Method]

In the plant cultivation method of the present embodiment, a sequence of irradiating a plant with solar light (hereinafter, also referred to as "solar light irradiation step"), a sequence of irradiating the plant with red light (hereinafter, also referred to as "red light irradiation step"), and a sequence of irradiating the plant with blue light (hereinafter, also referred to as "blue light irradiation step") are independently performed within a certain period of time using the plant cultivation apparatus of the present embodiment (Shigyo method).

In the sequence of irradiating a plant with red light in the present invention, irradiation light may include red light, and if the intensity of the red light included in the irradiation light is greater than or equal to 60%, the irradiation light may include light other than red light, for example, blue light. According to the findings of the inventor of the present application, the process of irradiating a plant with red light in the Shigyo method allows mixing of the red light with about 30% of blue light in an intensity ratio, and thus, it is possible to obtain an effect of enhancing growth of a plant within the allowable range. In order to enhance the effect of the Shigyo method, the mixed amount of the blue light is more preferably set to be less than or equal to 20% and most preferably set to be 0%. An example of the irradiation light intensity ratio in the sequence of irradiating a plant with red light includes 60% of red light, 20% of far infrared light, and 20% of blue light, and the most preferable intensity ratio is 100% of red light.

The intensity ratio of the irradiation light in the present invention is obtained using the photosynthetic photon flux density (PPFD, unit: µmol/m²s).

In the sequence of irradiating a plant with blue light in the present invention, irradiation light may include blue light, and if the intensity of the blue light included in the irradiation light is greater than or equal to 60%, the irradiation light may include light excluding the blue light, for example, red light. According to the findings of the inventor of the present application, the process of irradiating a plant with blue light in the Shigyo method allows mixing of the blue light with 30% of red light in an intensity ratio and it is possible to obtain an effect for promoting growth of a plant within the allowable range. In order to enhance the effect of the Shigyo method, the mixed amount of the red light is more preferably set to be less than or equal to 20% and most preferably set to be 0%. An example of the irradiation light intensity ratio in the sequence of irradiating a plant with blue light includes 60% of blue light, 20% of far infrared light, and 20% of red light, and the most preferable intensity ratio is 100% of blue light.

In the present embodiment, a case where the solar light irradiation step and a sequence of irradiating a plant with artificial light when the solar light is insufficient are performed is described as an example of the plant cultivation method of the present invention. In the sequence of irradiating the plant with artificial light, a red light irradiation step and a blue light irradiation step are independently performed within a certain period of time. In a case where the sequence of irradiating a plant with artificial light is performed when the solar light is insufficient, it is possible to enhance the energy efficiency, which is preferable.

The term "certain period of time" in the present embodiment means a period of an arbitrary length of time during plant cultivation. The period is the whole cultivation period at longest. In addition, the shortest period can be arbitrarily set as long as the present invention exhibits an effect. In this period, the unit of the length of time may be an hour (hr) basis, or may be a longer unit (for example, a day (day) basis) or a shorter unit (for example, a minute (minutes) basis). Flashing irradiation with a high frequency of greater than or equal to 1 Hz is not included in the scope "certain period of time" of the present invention, and is preferably within a range between 3 hours and 48 hours.

In addition, the term "independently" means that there is a red light irradiation step and a blue light irradiation step separately in the sequence of irradiating a plant with artificial light within the period described above.

At least one process between the solar light irradiation step, and the red light irradiation step and the blue light irradiation step included in the sequence of irradiating a plant with artificial light may be included in the period described above. The number of times of, the time of, and the order of performing the solar light irradiation step, the red light irradiation step, and the blue light irradiation step are appropriately selected depending on the type of the plant to be grown and the length of time (for example, nighttime, evening, or early morning) when the solar light is insufficient. It is preferable to select as to what number of times of, what time of, or what order of performing the steps a plant to be grown prefers, through experiments.

For example, the solar light irradiation step and the sequence of irradiating a plant with artificial light may be alternatingly performed in a continuous manner or may be discontinuously repeated by having a sequence of simultaneously irradiating a plant with solar light and artificial light or a sequence of suspending light irradiation on a plant, each sequence being interposed between both the steps. In addition, in the sequence of irradiating a plant with artificial light, red light irradiation step and the blue light irradiation step may be alternatingly performed in a continuous manner or may be discontinuously repeated by having a sequence of simultaneously irradiating a plant with red light and blue light or a sequence of suspending light irradiation on a plant, each sequence being interposed between both the steps.

In addition, for example, when starting the sequence of irradiating a plant with artificial light because of insufficient solar light at sunset after the solar light irradiation step during daytime, it is determined as to whether to perform the red light irradiation step or the blue light irradiation step subsequent to the solar light irradiation step based on the wavelength of light preferred by the plant. That is, the red light irradiation step may be performed or the blue light irradiation step may be performed subsequent to the solar light irradiation step.

For example, the evening sun is in a state where blue light is reduced, and thus, the blue light irradiation step may be performed. Similarly, the morning sun is also in a state where blue light is reduced, and thus, the blue light irradiation step may be performed.

In addition, for example, when starting the solar light irradiation step by the solar light sufficiently irradiating a plant due to sunrise after finishing the sequence of irradiating the plant with artificial light at night and then in the morning, it is determined as to whether to perform the red light irradiation step or the blue light irradiation step as a sequence of irradiating the plant with artificial light prior to the solar light irradiation step based on the wavelength of light preferred by the plant. That is, the solar light irradiation step may be performed subsequently to the red light irradiation step, or the solar light irradiation step may be performed subsequently to the blue light irradiation step.

In addition, the plant cultivation method of the present embodiment includes at least one process between the solar light irradiation step, and the red light irradiation step and the blue light irradiation step included in the sequence of irradiating a plant with artificial light, one or more irradiation cycle(s) where the predetermined number of times of, the time of, and the order of performing each step is/are determined and the cycle(s) may be performed one or more times. The time required for one irradiation cycle is the whole cultivation period at longest and can be arbitrarily set as long as the present invention exhibits an effect. In addition, in the plant cultivation method of the present embodiment, the number of irradiation cycles and the number of times the irradiation cycle is performed can be appropriately determined depending on the type of plant to be grown and are not particularly limited.

For example, in a case where one day is set as an irradiation cycle, it is possible to set the solar light irradiation step to be 10 hours and the sequence of irradiating a plant with artificial light to be 14 hours. In this case, in the sequence of irradiating a plant with artificial light, it is possible to set the red light irradiation step to be 7 hours and the blue light irradiation step to be 7 hours. In addition, in the sequence of irradiating a plant with artificial light, the red light irradiation step for 3.5 hours may be set to be performed twice and the blue light irradiation step for 3.5 hours may be set to be performed twice.

Regarding the wavelength of the red light and the blue light in the sequence of irradiating a plant with artificial light, in a case where a plurality of times of red light irradiation step and/or blue light irradiation step are performed, the wavelength in an N-th irradiation step CN (N is an integer of 1 or more) and the wavelength in an M-th irradiation step CM (M is an integer of 1 or more and is different from N) may be different from each other and may be same as each other.

In addition, in the sequence of irradiating a plant with artificial light, irradiation of light in a plurality of wavelength ranges may be performed by combining light in other wavelength ranges in addition to the red light and the blue light.

The light emission intensity of red light and/or blue light with which a plant is irradiated in the sequence of irradiating a plant with artificial light is not particularly limited, but is about 1 µmol/m²s to 1000 µmol/m²s, preferably about 10 µmol/m²s to 500 µmol/m²s, and particularly preferably about 50 µmol/m²s to 250 µmol/m²s by the photosynthetic photon flux density (PPFD).

In addition, the light intensity ratio of red light to blue light can be arbitrarily set as 1:1, 2:1, 4:1, 10:1, 20:1, or the like at "red : blue" or "blue : red", for example.

In the whole period of time of plant cultivation immediately after germinating seeds or immediately after setting out seedlings until harvest, the plant cultivation method according to the present invention can start or finish at an arbitrary timing and can be applied for an arbitrary length of time.

Plants cultivated using the plant cultivation method according to the present invention are not particularly limited, but examples thereof include vegetables, potatoes, fruits, pulses, grains, nuts, algae, ornamental plants, and mosses.

Examples of leafy vegetables include lettuce, salad vegetables, Glebionis coronaria, parsley, Japanese chervil, Japanese mustard spinach, mizuna greens, mustard mizuna greens, mustard, wasabi greens, watercress, Chinese cabbage, pickled greens, bok choy, cabbage, cauliflower, broccoli, brussels sprouts, onion, spring onion, sprouted Welsh onion, garlic, shallots, leeks, asparagus, celery, spinach, Japanese parsley, udo, Japanese ginger, butterbur, perilla, and various herbs. In addition, examples of leafy vegetables also include detroit, lollo rossa, arugula, pinot green, red romaine, and chicory that are called "baby leaf" and are mostly eaten as young leaves.

Examples of lettuce include head-forming lettuce, non-head-forming lettuce, and half-head-forming lettuce, such as leaf lettuce, frill lettuce, romaine, green wave, green leaf, red leaf, Frillice (registered trademark), River Green (registered trademark), frill leaf, fringe green, lettuce strong against tip burn, moco lettuce, Korean lettuce, and chimasanchu.

Examples of various herbs include basil and Italian parsley.

In addition, fruit vegetables such as tomatoes, melons, cucumber, strawberries, squashes, watermelons, eggplants, green peppers, okra, green beans, broad beans, peas, green soybean, or corn, and root vegetables such as radish, turnip, burdock, carrot, potato, taro, sweet potato, yam, ginger, wasabi, or lotus root can be cultivation targets.

Examples of fruit trees include mango, pineapple, figs, blueberries, raspberries, blackberries, boysenberries, grapes, nanking cherries, cranberries, blue honeysuckle, currants, cultivated currant, papaya, passion fruits, and dragon fruits.

Examples of grains include amaranthus, millet, oats, barley, proso millet, wheat, rice, glutinous rice, buckwheat, corn, pearl barley, barnyard millet, and rye.

Examples of algae include seaweed, that is, Laminaria japonica, Laminaria ochotensis, Laminaria diabolica, Laminaria longipedalis, Laminaria angustata, Laminaria longissima, Laminaria cichorioides, Laminaria sachalinensis, Laminaria coriacea, Kjellmaniella crassifolia, Cymathaere japonica, Arthrothamnus bifidus, Laminaria yezoensis, Alaria crassifolia, Cystoseira hakodatensis, Eisenia arborea, Eisenia bicyclis, Ecklonia cava, Ecklonia stolonifera, Sargassum fulvellum, Hizikia fusiformis, tubinariaornata, Hormophysa cuneiformis, Sargassum macrocarpum, Sargassum hemiphyllum, and Sargassum thunbergii. In addition, examples of algae also include seaweed that is called Caulerpa lentillifera and belongs to Family Caulerpa and Genus Caulerpa.

Examples of microalgae include algae belonging to Class Chlorophyceae or Class Trebouxiophyceae. Examples of Class Chlorophyceae include Botryococcus braunii and examples of Class Trebouxiophyceae include Psudochoricystis ellipsoidea. Since the Botryococcus braunii and the Psudochoricystis ellipsoidea produce heavy oil or light oil by photosynthesis, they are expected to be used for biofuel.

Examples of mosses include mosses belonging to Class Bryopsida. For example, there are mosses called Racomitriumjaponicum or Sunagoke mosses and belonging to Order Grimmiales, Family Grimmiaceae, and Genus Racomitrium.

In addition, as ornamental plants, various foliage plants in addition to rose, miniature rose, and gentian can be cultivation targets.

Since the plant cultivation method of the present embodiment is a method of independently performing a sequence of irradiating a plant with solar light, a sequence of irradiating a plant with red light and a sequence of irradiating a plant with blue light within a certain period of time, it is possible to promote the growth of a plant by irradiating a plant with the artificial light and the solar light and to obtain excellent energy efficiency.

More specifically, since the plant cultivation method of the present embodiment includes a sequence of irradiating a plant with solar light and a sequence of irradiating a plant with artificial light including red light and/or blue light, it is possible to promote the growth of a plant compared to a case where the artificial light is not used and to obtain excellent energy efficiency.

Moreover, in the plant cultivation method of the present embodiment, the sequence of irradiating a plant with red light and the sequence of irradiating a plant with blue light are independently performed within a certain period of time. Therefore, it is possible to simultaneously or alternatingly irradiate a plant with red light and blue light depending on the plant to be grown so as to obtain a sufficient effect for promoting the growth of the plant, to change the irradiation time of the red light and the blue light, and to easily irradiate a plant to be grown with optimum artificial light, thereby obtaining an excellent effect for promoting the growth of the plant.

In addition, the plant cultivation apparatus of the present embodiment includes a region in which a plant is irradiated with solar light; a light irradiation unit that irradiates a plant with artificial light including red light and/or blue light; and a control unit that controls the light irradiation unit to independently perform a step of irradiating a plant with red light and a step of irradiating the plant with blue light within a certain period of time. Therefore, it is possible to cultivate a plant using artificial light including red light and/or blue light and solar light and to easily irradiate a plant to be grown with optimum artificial light, to thereby promote the growth of a plant and to obtain excellent energy efficiency.

In addition, it is possible to obtain further excellent energy efficiency in a case where the plant cultivation method includes a sequence of irradiating a plant with solar light and a sequence of irradiating the plant with artificial light when the solar light is insufficient, in which in the sequence of irradiating the plant with artificial light, a sequence of irradiating the plant with red light, and a sequence of irradiating the plant with blue light are independently performed using the plant cultivation apparatus of the present embodiment within a certain period of time.

The present invention is not limited to the above-described embodiment.

For example, in the above-described embodiment, an example of a case where a plant is irradiated with the artificial light including red light and blue light when the solar light is insufficient is described, but the sequence of irradiating a plant with the artificial light may be performed when the solar light is sufficient. Specifically, the growth of a plant to be cultivated may be promoted by, for example, irradiating a plant with artificial light including red light and blue light in a state where part or all of the solar light is blocked so as to set light, with which the plant is irradiated, to be light preferred by the plant to be cultivated.

### [Example]

In the following Example, 6 seeds of an objective plant to observe a growth state were sown in a peat plate for growing at regular intervals and were germinated under a fluorescent light (12-hour day length). All the test groups were placed under an identical light environment for 3 days from the sowing until germinating.

Thereafter, the objective plants were placed and grown in cultivation rooms under different light environments of Examples 1 to 4 and Comparative Examples 1 to 6. All the environments within the cultivation rooms used in Examples 1 to 4 and Comparative Examples 1 to 6 were set to identical conditions at 25°C to 27°C of the atmospheric temperature and 50% humidity, except for the light irradiation condition. A cultivation room that has a wall and a ceiling formed of a sheet-shaped translucent resin and that can control the temperature and the humidity inside the room to be in a predetermined range was used. In addition, the carbonic acid gas concentration inside the cultivation room was controlled to be 1000 ppm.

### (Example 1)

In Example 1, leaf lettuce (variety: Summer surge) was used as an objective plant to observe a growth state.

In Example 1, a light irradiation unit having red light-emitting elements formed of 180 red LEDs (center wavelength: 660 nm, HRP-350F made by SHOWA DENKO K.K.) and having blue light-emitting elements formed of 60 blue LEDs (center wavelength: 450 nm, GM2LR450G made by SHOWA DENKO K.K.) was used. Moreover, a control unit that controls the light irradiation unit to independently perform a step of irradiating a plant only with red light and a step of irradiating the plant only with blue light within a certain period of time was used.

The photosynthetic photon flux density (PPFD) of red light as the light emission intensity of red light from the light irradiation unit was set as 225 µmol/m²s and the photosynthetic photon flux density (PPFD) of blue light from the light irradiation unit was set as 75 µmol/m²s (light emission intensity ratio of the red light to the blue light is 3:1).

Moreover, solar light was introduced to the inside of the cultivation room through the wall and ceiling formed of translucent material from 5 a.m. to 7 p.m. (for 14 hours) and the control unit controlled the light irradiation unit to perform a step of irradiating the plant only with red light from 7 p.m. for 5 hours and then a step of irradiating the plant only with blue light until 5 a.m. next morning (for 5 hours). During the time from 7 p.m. until 5 a.m. next morning, the PPFD of the solar light was in a state of less than or equal to 50 µmol/m²s.

### (Example 2)

The same test as Example 1 was performed except for performing a step of irradiating a plant only with blue light for 5 hours from 7 p.m. and then a step of irradiating the plant only with red light until 5 a.m. next morning (for 5 hours).

### (Example 3)

The same test as Example 1 was performed except that sprouted Welsh onion was used as an objective plant to observe a growth state and the photosynthetic photon flux density (PPFD) of red light as the light emission intensity of red light from the light irradiation unit was set as 120 µmol/m²s and the photosynthetic photon flux density (PPFD) of blue light from the light irradiation unit was set as 60 µmol/m²s (light emission intensity ratio of the red light to the blue light is 2:1).

### (Example 4)

The same test as Example 2 was performed except that sprouted Welsh onion was used as an objective plant to observe a growth state and the photosynthetic photon flux density (PPFD) of red light as the light emission intensity of red light from the light irradiation unit was set as 120 µmol/m²s and the photosynthetic photon flux density (PPFD) of blue light from the light irradiation unit was set as 60 µmol/m²S (light emission intensity ratio of the red light to the blue light is 2:1).

### (Comparative Example 1)

The same test as Example 1 was performed except that a plant was not irradiated with artificial light.

### (Comparative Example 2)

The same test as Example 1 was performed except that a red LED and a blue LED were turned on from 7 p.m. until 5 a.m. (for 10 hours) and the plant was simultaneously irradiated with the red light and the blue light.

### (Comparative Example 3)

The same test as Example 3 was performed except that a plant was not irradiated with artificial light.

### (Comparative Example 4)

The same test as Example 3 was performed except that a red LED and a blue LED were turned on from 7 p.m. until 5 a.m. (for 10 hours) and the plant was simultaneously irradiated with the red light and the blue light.

### (Comparative Example 5)

The same test as Example 1 was performed except that a plant was not irradiated with solar light.

### (Comparative Example 6)

The same test as Example 3 was performed except that a plant was not irradiated with solar light.

As to the plants grown for 24 days in Examples 1 and 2 and Comparative Examples 1, 2, and 5, the weight of the above-ground part (above-ground part fresh weight, fresh weight above-ground part) was measured to obtain a mass ratio by setting the mass ratio (fresh weight ratio above-ground part) of Example 1 as 100%.

In addition, as to the plants grown for 14 days in Examples 3 and 4 and Comparative Examples 3, 4, and 6, the weight of the above-ground part (above-ground part fresh weight) was measured to obtain a mass ratio by setting the mass ratio of Example 3 as 100%.

In addition, the electric power used for growing the plants in Examples 1 to 4 and Comparative Examples 1 to 6 was measured. The result is shown in Table 1.

**TABLE. 1**

| | FRESH WEIGHT RATIO OF ABOVE-GROUND PART (%) | POWER CONSUMPTION(kWh) |
|---|---|---|
| EXAMPLE 1 | 100 | 9.6 |
| EXAMPLE 2 | 95 | 9.6 |
| EXAMPLE 3 | 100 | 3.3 |
| EXAMPLE 4 | 110 | 3.3 |
| COMPARATIVE EXAMPLE 1 | 45 | 0 |
| COMPARATIVE EXAMPLE 2 | 68 | 19.2 |
| COMPARATIVE EXAMPLE 3 | 55 | 0 |
| COMPARATIVE EXAMPLE 4 | 73 | 6.6 |
| COMPARATIVE EXAMPLE 5 | 40 | 9.6 |
| COMPARATIVE EXAMPLE 6 | 38 | 3.3 |

As shown in Table 1, it was found that Examples 1 and 2 in which the sequence of irradiating a plant with solar light, the sequence of irradiating a plant with red light, and the sequence of irradiating a plant with blue light were independently performed within a certain period of time exhibit a strong effect for promoting growth of a plant in comparison with Comparative Example 2 in which a plant was simultaneously irradiated with red light and blue light. Moreover, it was also found that the used electric power (power consumption) was reduced to a half in Examples 1 and 2 in comparison with Comparative Example 2 thereby obtaining remarkably excellent energy efficiency.

In addition, it was found that Examples 1 and 2 exhibit a strong effect for promoting growth of a plant in comparison with Comparative Example 1 in which a plant was not irradiated with artificial light.

In addition, it was found that Examples 1 and 2 exhibit a strong effect for promoting growth of a plant in comparison with Comparative Example 5 in which a plant was not irradiated with solar light, although the power consumption in Example 1 and 2 are the same as that in Comparative Example 5.

In addition, it was found that, in a case of using the leaf lettuce as shown in Example 1, the effect for promoting growth of the plant was high when performing the step of irradiating the plant with solar light, the step of irradiating the plant with red light, the step of irradiating the plant with blue light, and then the step of irradiating the plant with solar light in comparison with Example 2 in which the step of irradiating a plant with red light and the step of irradiating a plant with blue light were performed in an opposite order to Example 1.

As mentioned above, it was found that there are plants which exhibit a strong effect for promoting growth by performing the sequence of irradiating the plant with sunlight, the sequence of irradiating the plant with red light, and the sequence of irradiating the plant with blue light in order. As the plant, for example, there are perilla, leeks, Japanese chervil, and watercress, other than leaf lettuce.

In addition, as shown in Table 1, it was found that Examples 3 and 4 in which the sequence of irradiating a plant with solar light, the sequence of irradiating a plant with red light, and the sequence of irradiating a plant with blue light were independently performed within a certain period of time exhibit a high effect for promoting growth of a plant in comparison with Comparative Example 4 in which a plant was simultaneously irradiated with red light and blue light. Moreover, it was also found that the used electric power was reduced to a half in Examples 3 and 4 in comparison with Comparative Example 4 thereby obtaining remarkably excellent energy efficiency.

In addition, it was found that Examples 3 and 4 exhibit a high effect for promoting growth of a plant in comparison with Comparative Example 3 in which a plant was not irradiated with artificial light.

In addition, it was found that Examples 3 and 4 exhibit a high effect for promoting growth of a plant in comparison with Comparative Example 6 in which a plant was not irradiated with solar light, although the power consumption in Example 3 and 4 are the same as that in Comparative Example 6.

In addition, it was found that, in a case of using the sprouted Welsh onion as shown in Example 4, the effect for promoting growth of the plant was high when performing the step of irradiating the plant with solar light, the step of irradiating the plant with blue light, the step of irradiating the plant with red light, and then the step of irradiating the plant with solar light in comparison with Example 3 in which the step of irradiating a plant with red light and the step of irradiating a plant with blue light were performed in an opposite order to Example 4.

That is, from Examples 1 to 4, it was found that the relationship between the order of performing the step of irradiating a plant with red light and the step of irradiating a plant with blue light and the effect for promoting the growth of the plant varies depending on the type of plant.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

As mentioned above, it was found that there are plants which exhibit a strong effect for promoting growth by performing the sequence of irradiating the plant with sunlight, the sequence of irradiating the plant with blue light, and the sequence of irradiating the plant with red light in order. As the plant, for example, there are tomatoes, strawberries, melons, green peppers, and okra, other than sprouted Welsh onion.

## Claims

1. A plant cultivation method comprising:
a sequence of irradiating a plant with sunlight;
a sequence of irradiating the plant with red light; and
a sequence of irradiating the plant with blue light,
wherein the sequences are performed independently within a certain period of time.

2. The plant cultivation method according to Claim 1,
wherein the sequence of irradiating the plant with red light is performed subsequently to the sequence of irradiating the plant with sunlight.

3. The plant cultivation method according to Claim 1,
wherein the sequence of irradiating the plant with blue light is performed subsequently to the sequence of irradiating the plant with sunlight.

4. The plant cultivation method according to Claim 1,
wherein the sequence of irradiating the plant with sunlight is performed subsequently to the sequence of irradiating the plant with red light.

5. The plant cultivation method according to Claim 1,
wherein the sequence of irradiating the plant with sunlight is performed subsequently to the sequence of irradiating the plant with blue light.

## Patentansprüche

1. Pflanzenkultivierungsverfahren, welches umfasst:
eine Sequenz einer Belichtung einer Pflanze mit Sonnenlicht;
eine Sequenz einer Belichtung der Pflanze mit rotem Licht; und
eine Sequenz einer Belichtung der Pflanze mit blauem Licht, wobei die Sequenzen innerhalb eines bestimmten Zeitraums unabhängig voneinander durchgeführt werden.

2. Pflanzenkultivierungsverfahren nach Anspruch 1, wobei die Sequenz der Belichtung der Pflanze mit rotem Licht nach der Sequenz der Belichtung der Pflanze mit Sonnenlicht durchgeführt wird.

3. Pflanzenkultivierungsverfahren nach Anspruch 1, wobei die Sequenz der Belichtung der Pflanze mit blauem Licht nach der Sequenz der Belichtung der Pflanze mit Sonnenlicht durchgeführt wird.

4. Pflanzenkultivierungsverfahren nach Anspruch 1, wobei die Sequenz der Belichtung der Pflanze mit Sonnenlicht nach der Belichtung der Sequenz der Belichtung der Pflanze mit rotem Licht durchgeführt wird.

5. Pflanzenkultivierungsverfahren nach Anspruch 1, wobei die Sequenz der Belichtung der Pflanze mit Sonnenlicht nach der Sequenz der Belichtung der Pflanze mit blauem Licht durchgeführt wird.

## Revendications

1. Procédé de culture de plantes, comprenant:
une séquence d'irradiation d'une plante avec la lumière solaire;
une séquence d'irradiation de la plante avec une lumière rouge; et
une séquence d'irradiation de la plante avec une lumière bleue,
dans lequel les séquences sont exécutées de façon indépendante à l'intérieur d'une certaine période de temps.

2. Procédé de culture de plantes selon la revendication 1, dans lequel la séquence d'irradiation de la plante avec une lumière rouge est exécutée à la suite de la séquence d'irradiation de la plante avec la lumière solaire.

3. Procédé de culture de plantes selon la revendication 1, dans lequel la séquence d'irradiation de la plante avec une lumière bleue est exécutée à la suite de la séquence d'irradiation de la plante avec la lumière solaire.

4. Procédé de culture de plantes selon la revendication 1, dans lequel la séquence d'irradiation de la plante avec la lumière solaire est exécutée à la suite de la séquence d'irradiation de la plante avec une lumière rouge.

5. Procédé de culture de plantes selon la revendication 1, dans lequel la séquence d'irradiation de la plante avec la lumière solaire est exécutée à la suite de la séquence d'irradiation de la plante avec une lumière bleue.
